# EUROPEAN PATENT APPLICATION

(11) **EP 4 579 677 A1**
(43) Date of publication of application: **02.07.2025**
(21) Application number: 24223532.3
(22) Date of filing: 27.12.2024
(51) Int. Cl.: G16H 20/40, A61G 13/02, G16H 40/63

(54) **SURGICAL TABLE MONITORING AND ALERTING SYSTEM**

(30) Priority: 28.12.2023 US 202363615753 P
(71) Applicant: Stryker Corporation, Portage, MI 49002-9711 (US)
(72) Inventor: WILLIAMS III, Thomas C., Portage, 49002 (US)
(74) Representative: V.O.

(57) **Abstract**

The present disclosure relates generally to improving surgery safety, and more specifically to techniques for monitoring the position of patients on surgical tables. An exemplary computer-implemented method for outputting warnings related to a patient on a surgical table during a surgical procedure comprises: determining a configuration of the surgical table; determining, based on the configuration of the surgical table, that the patient is in an at-risk position; initiating a timer to track an elapsed time period during which the patient is in the at-risk position; determining that the elapsed time period exceeds a predefined time threshold; and outputting a warning related to the patient on the surgical table.

## Description

### CROSS-REFERENCE TO RELATED APPLICATIONS

This application claims the benefit of U.S. Provisional Patent Application No. 63/615,753, filed December 28, 2023, the entire contents of which is hereby incorporated by reference herein.

### FIELD

The present disclosure relates generally to improving surgery safety, and more specifically to techniques for automatically monitoring the position of patients on surgical tables and providing intelligent alerts and/or interventions.

### BACKGROUND

Ensuring the safety and health of patients during surgery is a major focus area for hospitals. During a surgical procedure, a patient may be placed on a surgical table in various positions to allow medical practitioners (e.g., surgeons, nurses, and other members of the surgical staff) to access and operate on various parts of the patient's body. However, certain positions, if maintained for an extended duration, may negatively impact the patient's health during and/or after the surgical procedure. For example, maintaining a Trendelenburg position, in which the surgical table is tilted such that the patient's head is lower than the patient's feet, for an extended duration may lead to physiological changes including increased venous return, increased intra-cranial and cardiac pressure, ocular edema, and respiratory complications. These physiological changes, in turn, may increase the risk of surgical complications for the patient. To reduce the possibility of complications, medical organizations recommend that the Trendelenburg position be used for the shortest length of time possible and that the surgical table be returned to a horizontal or head-up position at specific intervals during a lengthy surgical procedure. This is especially important when the angle of the surgical table is steep (e.g., 30 degrees or greater).

Currently, no automated systems are provided to monitor the position of patients on a surgical table and alert medical practitioners to take appropriate actions (e.g., repositioning the patient) when the patient has been in an at-risk position for an extended duration. Instead, medical practitioners are responsible for manually keeping track of how long a patient has been placed in an at-risk position and making ad-hoc decisions regarding whether and/or when to reposition the patient. This imposes an increased cognitive burden on medical practitioners in a high-stress environment and may lead to inconsistent practices, thereby compromising patient safety.

### SUMMARY

Disclosed herein are exemplary devices, apparatuses, systems, methods, and non-transitory storage media for monitoring various aspects of the position of patients on surgical tables and providing warnings and/or intervention mechanisms in an intelligent manner. An at-risk position may include any patient position that can increase the risk of surgical complications. For example, the at-risk position may include a Trendelenburg position, in which the surgical table is tilted such that the patient's head is lower than the patient's feet, due to the negative health consequences of increasing blood flow to the patient's head, increasing intraocular pressure, and/or increasing intracranial pressure for an extended duration. The at-risk position may include other tilt positions, for example, when the surgical table is tilted such that any part of the patient's body is elevated relative to the other parts, due to the increased risk of muscle and nerve injuries associated with elevating the body part for an extended duration. In some examples, the at-risk position involves an orientation of the patient. For example, the at-risk position may include a prone position, in which the surgical table is not tilted and the patient's abdomen is flush against the surgical table, due to the negative health consequences of applying pressure to the patient's abdomen for an extended duration. The at-risk position may include a traction position, in which mechanical tension is applied to various parts of the patient's body using a traction mechanism, due to the increased risk of damage to tissues surrounding the traction mechanism.

In some examples, the system may monitor the patient's position on the surgical table by determining the configuration of the surgical table. In some examples, the configuration of the surgical table may include an angle between the table surface of the surgical table and a horizontal level (referred to herein as "the angle of the surgical table"). In some examples, the angle of the surgical table may be retrieved from a memory unit associated with the surgical table. In some examples, the angle of the surgical table may be determined based on previous user input(s), such as a user's button presses on a remote controller to tilt the table. In some examples, the angle of the surgical table may be determined based on one or more sensors. In some examples, the angle of the surgical table may be determined based on a machine-learning model. For example, by analyzing one or more images of the surgical environment (e.g., captured by one or more cameras in the surgical environment), the machine-learning model can identify the configuration of the surgical table (e.g., the angle of the surgical table, relative locations of the patient's feet and head, and the like).

Based on the angle between the table surface of the surgical table and a horizontal level, the system may determine that the patient is in a Trendelenburg position and/or another at-risk tilt position. To do this, the system may compare the angle between the table surface of the surgical table and the horizontal level to a predefined angle threshold. If the angle exceeds the predefined angle threshold, the patient is determined to be in the Trendelenburg position and/or another at-risk tilt position.

In some examples, the configuration of the surgical table comprises the direction that the patient is facing on the surgical table (e.g., whether the patient is lying face-down and the patient's abdomen is flush against the surgical table) and/or which body parts are in contact with the surgical table. In some examples, the system may determine the direction that the patient is facing based on readings from one or more pressure sensors of the surgical table. In some examples, the system may make the determination using a machine-learning model. By analyzing one or more images of the surgical environment, the machine-learning model can identify the position of the patient (e.g., body parts of the patient, such as head, face, chest, abdomen, back, waist, hips, legs, knees, and feet of the patient, and/or their directions and relative positions to each other). In some examples, the system may make the determination based on one or more user inputs (e.g., a user input indicative that the patient has been placed in the prone position).

In some examples, the configuration of the surgical table comprises active mechanisms of the surgical table (e.g., a traction mechanism). In some examples, mechanical tension may be applied to one or more body parts of the patient via a traction mechanism. One or more force sensors of the surgical table may obtain tension measurements indicative of traction. Based on the tension measurements, the system may determine that the patient is in a traction position and is thus in an at-risk position. In some examples, the system may make the determination using a machine-learning model. By analyzing one or more images of the surgical environment, the machine-learning model can identify traction mechanisms applied to the patient.

After the patient is determined to be in an at-risk position based on the configuration of the surgical table, the system may initiate a timer to track an elapsed time period during which the patient is in the at-risk position. The system may further determine that the elapsed time period exceeds a predefined time threshold, which may be the maximum duration of time that a patient can spend in an at-risk position without risk of surgical complications. The predefined time threshold may be predefined according to medical recommendations, predefined by a manufacturer of the surgical table, and/or predefined by a user of the table (e.g., a medical practitioner). In some examples, the predefined time threshold may be determined based on various factors, such as the patient's age and medical characteristics, historical data associated with the patient and the surgical procedure, and/or characteristics of the surgeon performing the surgical procedure. In some examples, the system may use one or more machine-learning models and/or statistical techniques to draw correlations between the various factors described above to determine the predefined time threshold. For example, based on historical timing data from a plurality of surgical procedures, a prediction algorithm of the system may determine that a specific surgical procedure will have the greatest chance of success if the patient spends less than 30 minutes in a tilt position. Accordingly, the system may determine that the predefined time threshold should be set to 30 minutes.

The system may further output a warning related to the patient on the surgical table. For example, the system may first output a warning that the patient has been placed in an at-risk position for a duration exceeding the predefined time threshold and/or request that the surgical table be repositioned. If a warning is issued and the patient remains in the at-risk position, the system may eventually issue another warning and/or provide automatic intervention (e.g., returning the surgical table to a non-tilted position). The automatic intervention may require preparation to ensure patient safety during the intervention (e.g., the medical practitioner may restrain the patient on the surgical table and/or clear the surroundings of hazards). As such, additional warnings may be provided in advance of the automatic intervention to give the user sufficient notice and/or an opportunity to opt out of automatic intervention. For example, if the patient remains in the at-risk position for a second duration exceeding a second predefined time threshold (e.g., longer than the first predefined time threshold), the system may follow up with a second warning and/or automatically return the surgical table to the horizontal level position if the medical practitioner does not respond to the second warning. In some examples, the system may be configurable to allow medical practitioners to configure the system to disable automatic interventions.

In some examples, the system may display the elapsed time period and/or the various warnings on a display in the medical care area (e.g., the operating room). The system may display the elapsed time period and/or the warnings on a display in a monitoring area (e.g., at a central control center for monitoring multiple medical care areas). The system may display the elapsed time period and/or the warnings as messages (e.g., a text message, a notification) on a mobile device. The elapsed time period and/or the warning may comprise a visual component, an audio component, a haptic component, or any combination thereof. In some examples, the elapsed time period and/or the warning may be provided based on user-configurable settings (e.g., at user-specified frequency).

In some examples, some or all of the parameters and analytics described herein may be displayed on a display inside or outside the medical care area, including: the configuration of the surgical table, the duration in which the surgical table has been in a given configuration (e.g., tilted at or greater than a given angle), predefined angle thresholds (e.g., angles associated with at-risk positions), predefined time thresholds (e.g., durations associated with at-risk positions), the number of times that the surgical table has been repositioned, the average duration that the patient spends in a given position on the surgical table, etc. In some examples, the analytics may be conveyed in visual format, auditory format, haptic format, or any combination thereof.

The techniques described herein provide a number of technical advantages. For example, the techniques provide automated systems for monitoring patients and warning medical practitioners when patients have been placed in at-risk positions. This reduces the cognitive burden on medical practitioners when caring for and/or operating on patients during surgical procedures and ensures that patients receive appropriate care with greater consistency. The data gathered and analysis performed may be used to identify positive and negative trends associated with at-risk positions to improve medical practitioner practices and protocols and lead to better patient outcomes. In various examples, the techniques disclosed herein may improve functioning of a computer by efficient monitoring, analysis, and management of surgical procedures, reducing processing power, battery usage, and memory requirements associated with automatically monitoring the position of patients and providing intelligent alerts and/or interventions.

An exemplary computer-implemented method for outputting warnings related to a patient on a surgical table during a surgical procedure comprises: determining a configuration of the surgical table; determining, based on the configuration of the surgical table, that the patient is in an at-risk position; initiating a timer to track an elapsed time period during which the patient is in the at-risk position; determining that the elapsed time period exceeds a predefined time threshold; and outputting a warning related to the patient on the surgical table.

In some aspects, the at-risk position includes a Trendelenburg position, a prone position, a tilt position, a traction position, or any combination thereof. In some aspects, determining the configuration of the surgical table comprises determining if an angle between a table surface of the surgical table and a horizontal level is larger than a first predefined angle threshold.

In some aspects, the timer is a first timer and the method further comprises: while the patient is in the at-risk position, determining that the angle between the table surface of the surgical table and the horizontal level is larger than a second predefined angle threshold, the second predefined angle threshold larger than the first predefined angle threshold; and initiating a second timer.

In some aspects, the first predefined angle threshold is a value ranging from 15 to 29 degrees, and wherein the second predefined angle threshold is a value larger than 29 degrees. In some aspects, the predefined time threshold is determined based on a Body Mass Index (BMI) of the patient, an age of the patient, one or more pre-existing medical conditions of the patient, historical data associated with the patient, historical data associated with the surgical procedure, a milestone associated with the surgical procedure, a phase associated with the surgical procedure, or any combination thereof.

In some aspects, the predefined time threshold is determined based on a user profile associated with the surgical procedure. In some aspects, the predefined time threshold is determined based on a type of the surgical procedure. In some aspects, the predefined time threshold is determined based on a machine-learning model.

In some aspects, the method further comprises: displaying the elapsed time period and/or the warning on one or more displays. In some aspects, the elapsed time period is displayed in accordance with a color scheme to indicate the length of the elapsed time period. In some aspects, the one or more displays comprise a display on a control device communicatively coupled with the surgical table. In some aspects, the one or more displays comprise a surgical display, a surgical control panel, a dashboard, or any combination thereof. In some aspects, the warning is visual, auditory, haptic, or any combination thereof.

In some aspects, the method further comprises: determining that the elapsed time period exceeds the predefined time threshold; and causing output of a notification that the surgical table will automatically return to a horizontal level position. In some aspects, the notification includes a countdown. In some aspects, the predefined time threshold is a first predefined time threshold and the method further comprises: determining that the elapsed time period exceeds a second predefined time threshold, the second predefined time threshold longer than the first predefined time threshold; and causing output of a notification that the surgical table will automatically return to a horizontal level position. In some aspects, the notification includes a countdown.

In some aspects, the method further comprises receiving a user request to prevent the surgical table from automatically returning to the horizon level position; and upon receiving the user request, foregoing automatically returning the surgical table to the horizontal level position. In some aspects, the method further comprises: after outputting the notification, automatically returning the surgical table to the horizontal level position.

An exemplary system for outputting warnings during a surgical procedure comprises: one or more processors; a memory; and one or more programs, wherein the one or more programs are stored in the memory and configured to be executed by the one or more processors, the one or more programs including instructions for: determining a configuration of a surgical table accommodating a patient; determining, based on the configuration of the surgical table, that the patient is in an at-risk position; initiating a timer to track an elapsed time period during which the patient is in the at-risk position; determining that the elapsed time period exceeds a predefined time threshold; and outputting a warning related to the patient on the surgical table. The exemplary system can comprise the surgical table for accommodating the patient during the surgical procedure. An exemplary non-transitory computer-readable storage medium stores one or more programs, the one or more programs comprising instructions, which when executed by one or more processors of an electronic device, cause the electronic device to perform any of the techniques described herein. An exemplary computer program product comprises software code portions, which when executed by one or more processors of an electronic device, cause the electronic device to perform any of the techniques described herein.

It will be appreciated that any of the aspects, features and options described herein can be combined. It will particularly be appreciated that any of the aspects, features and options described in view of the methods apply equally to the systems, storage mediums and computer program products, and vice versa.

### BRIEF DESCRIPTION OF THE FIGURES

FIG. 1A illustrates an exemplary view of a medical care area in which a patient is placed in a Trendelenburg position on a surgical table, in accordance with various examples.
FIG. 1B illustrates a patient placed in a prone position on a surgical table, in accordance with various examples.
FIG. 1C illustrates a patient placed in a traction position on a surgical table, in accordance with various examples.
FIG. 1D illustrates a patient placed in a tilted position on a surgical table, in accordance with various examples.
FIG. 2 illustrates an exemplary process for generating a warning related to a patient in an at-risk position on a surgical table, in accordance with various examples.
FIG. 3 depicts an exemplary electronic device, in accordance with some examples.

### DETAILED DESCRIPTION

Disclosed herein are exemplary devices, apparatuses, systems, methods, and non-transitory storage media for monitoring various aspects of the position of patients on surgical tables and providing warnings and/or intervention mechanisms in an intelligent manner. An at-risk position may include any patient position that can increase the risk of surgical complications. For example, the at-risk position may include a Trendelenburg position, in which the surgical table is tilted such that the patient's head is lower than the patient's feet, due to the negative health consequences of increasing blood flow to the patient's head, increasing intraocular pressure, and/or increasing intracranial pressure for an extended duration. The at-risk position may include other tilt positions, for example, when the surgical table is tilted such that any part of the patient's body is elevated relative to the other parts, due to the increased risk of muscle and nerve injuries associated with elevating the body part for an extended duration. In some examples, the at-risk position involves an orientation of the patient. For example, the at-risk position may include a prone position, in which the surgical table is not tilted and the patient's abdomen is flush against the surgical table, due to the negative health consequences of applying pressure to the patient's abdomen for an extended duration. The at-risk position may include a traction position, in which mechanical tension is applied to various parts of the patient's body using a traction mechanism, due to the increased risk of damage to tissues surrounding the traction mechanism.

In some examples, the system may monitor the patient's position on the surgical table by determining the configuration of the surgical table. In some examples, the configuration of the surgical table may include an angle between the table surface of the surgical table and a horizontal level (referred to herein as "the angle of the surgical table"). In some examples, the angle of the surgical table may be retrieved from a memory unit associated with the surgical table. In some examples, the angle of the surgical table may be determined based on previous user input(s), such as a user's button presses on a remote controller to tilt the table. In some examples, the angle of the surgical table may be determined based on one or more sensors. In some examples, the angle of the surgical table may be determined based on a machine-learning model. For example, by analyzing one or more images of the surgical environment (e.g., captured by one or more cameras in the surgical environment), the machine-learning model can identify the configuration of the surgical table (e.g., the angle of the surgical table, relative locations of the patient's feet and head, and the like).

Based on the angle between the table surface of the surgical table and a horizontal level, the system may determine that the patient is in a Trendelenburg position and/or another at-risk tilt position. To do this, the system may compare the angle between the table surface of the surgical table and the horizontal level to a predefined angle threshold. If the angle exceeds the predefined angle threshold, the patient is determined to be in the Trendelenburg position and/or another at-risk tilt position.

In some examples, the configuration of the surgical table comprises the direction that the patient is facing on the surgical table (e.g., whether the patient is lying face-down and the patient's abdomen is flush against the surgical table) and/or which body parts are in contact with the surgical table. In some examples, the system may determine the direction that the patient is facing based on readings from one or more pressure sensors of the surgical table. In some examples, the system may make the determination using a machine-learning model. By analyzing one or more images of the surgical environment, the machine-learning model can identify the position of the patient (e.g., body parts of the patient, such as head, face, chest, abdomen, back, waist, hips, legs, knees, and feet of the patient, and/or their directions and relative positions to each other). In some examples, the system may make the determination based on one or more user inputs (e.g., a user input indicative that the patient has been placed in the prone position).

In some examples, the configuration of the surgical table comprises active mechanisms of the surgical table (e.g., a traction mechanism). In some examples, mechanical tension may be applied to one or more body parts of the patient via a traction mechanism. One or more force sensors of the surgical table may obtain tension measurements indicative of traction. Based on the tension measurements, the system may determine that the patient is in a traction position and is thus in an at-risk position. In some examples, the system may make the determination using a machine-learning model. By analyzing one or more images of the surgical environment, the machine-learning model can identify traction mechanisms applied to the patient.

After the patient is determined to be in an at-risk position based on the configuration of the surgical table, the system may initiate a timer to track an elapsed time period during which the patient is in the at-risk position. The system may further determine that the elapsed time period exceeds a predefined time threshold, which may be the maximum duration of time that a patient can spend in an at-risk position without risk of surgical complications. The predefined time threshold may be predefined according to medical recommendations, predefined by a manufacturer of the surgical table, and/or predefined by a user of the table (e.g., a medical practitioner). In some examples, the predefined time threshold may be determined based on various factors, such as the patient's age and medical characteristics, historical data associated with the patient and the surgical procedure, and/or characteristics of the surgeon performing the surgical procedure. In some examples, the system may use one or more machine-learning models and/or statistical techniques to draw correlations between the various factors described above to determine the predefined time threshold. For example, based on historical timing data from a plurality of surgical procedures, a prediction algorithm of the system may determine that a specific surgical procedure will have the greatest chance of success if the patient spends less than 30 minutes in a tilt position. Accordingly, the system may determine that the predefined time threshold should be set to 30 minutes.

The system may further output a warning related to the patient on the surgical table. For example, the system may first output a warning that the patient has been placed in an at-risk position for a duration exceeding the predefined time threshold and/or request that the surgical table be repositioned. If a warning is issued and the patient remains in the at-risk position, the system may eventually issue another warning and/or provide automatic intervention (e.g., returning the surgical table to a non-tilted position). The automatic intervention may require preparation to ensure patient safety during the intervention (e.g., the medical practitioner may restrain the patient on the surgical table and clear the surroundings of hazards). As such, additional warnings may be provided in advance of the automatic intervention to give the user sufficient notice and/or an opportunity to opt out of automatic intervention. For example, if the patient remains in the at-risk position for a second duration exceeding a second predefined time threshold (e.g., longer than the first predefined time threshold), the system may follow up with a second warning and/or automatically return the surgical table to the horizontal level position if the medical practitioner does not respond to the second warning. In some examples, the system may be configurable to allow medical practitioners to configure the system to disable automatic interventions.

In some examples, the system may display the elapsed time period and/or the various warnings on a display in the medical care area (e.g., the operating room). The system may display the elapsed time period and/or the warnings on a display in a monitoring area (e.g., at a central control center for monitoring multiple medical care areas). The system may display the elapsed time period and/or the warnings as messages (e.g., a text message, a notification) on a mobile device. The elapsed time period and/or the warning may comprise a visual component, an audio component, a haptic component, or any combination thereof. In some examples, the elapsed time period and/or the warning may be provided based on user-configurable settings (e.g., at user-specified frequency).

In some examples, some or all of the parameters and analytics described herein may be displayed on a display inside or outside the medical care area, including: the configuration of the surgical table, the duration in which the surgical table has been in a given configuration (e.g., tilted at or greater than a given angle), predefined angle thresholds (e.g., angles associated with at-risk positions), predefined time thresholds (e.g., durations associated with at-risk positions), the number of times that the surgical table has been repositioned, the average duration that the patient spends in a given position on the surgical table, etc. In some examples, the analytics may be conveyed in visual format, auditory format, haptic format, or any combination thereof.

The techniques described herein provide a number of technical advantages. For example, the techniques provide automated systems for monitoring patients and warning medical practitioners when patients have been placed in at-risk positions. This reduces the cognitive burden on medical practitioners when caring for and/or operating on patients during surgical procedures and ensures that patients receive appropriate care with greater consistency. The data gathered and analysis performed may be used to identify positive and negative trends associated with at-risk positions to improve medical practitioner practices and protocols and lead to better patient outcomes. In various examples, the techniques disclosed herein may improve functioning of a computer by efficient monitoring, analysis, and management of surgical procedures, reducing processing power, battery usage, and memory requirements associated with automatically monitoring the position of patients and providing intelligent alerts and/or interventions.

The following description sets forth exemplary methods, parameters, and the like. It should be recognized, however, that such description is not intended as a limitation on the scope of the present disclosure but is instead provided as a description of exemplary examples.

Although the following description uses terms "first," "second," etc. to describe various elements, these elements should not be limited by the terms. These terms are only used to distinguish one element from another. For example, a first graphical representation could be termed a second graphical representation, and, similarly, a second graphical representation could be termed a first graphical representation, without departing from the scope of the various described examples. The first graphical representation and the second graphical representation are both graphical representations, but they are not the same graphical representation.

The terminology used in the description of the various described examples herein is for the purpose of describing particular examples only and is not intended to be limiting. As used in the description of the various described examples and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

The term "if' is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

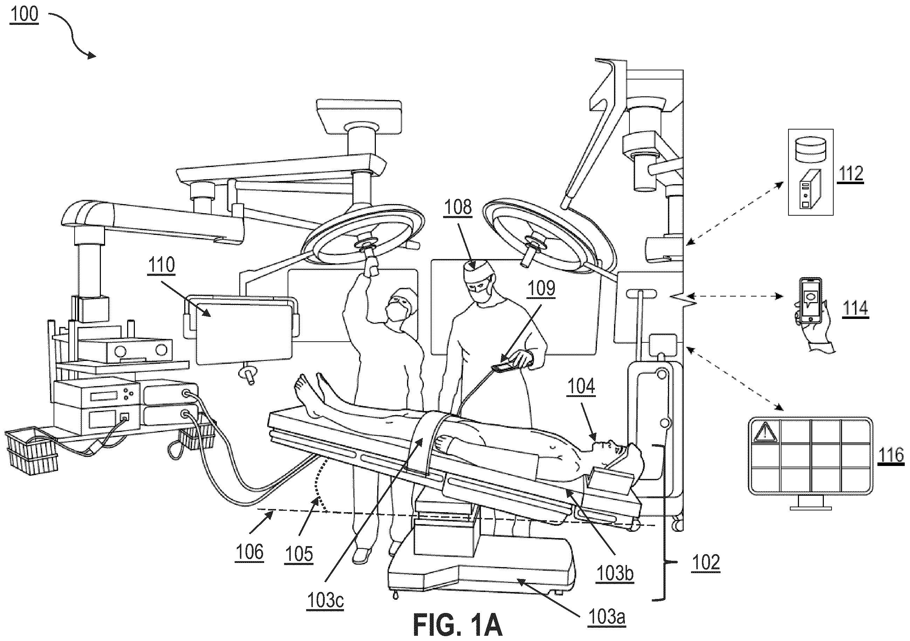

FIG. 1A illustrates an exemplary view of a medical care area 100, in accordance with some examples. In the illustrated example, the medical care area 100 is an operating room where surgical procedures can be performed. In some examples, the medical care area 100 may be part of a medical facility, such as a hospital, which includes a central control room for monitoring multiple medical care areas. The medical care area 100 may be a sterile environment isolated from other parts of the medical facility. The medical care area 100 may contain one or more portions of a system for monitoring patients and warning medical practitioners when patients have been placed in at-risk positions, as described herein.

The medical care area 100 can include a surgical table 102. In the illustrated example, the surgical table 102 has a base 103a supported by a floor of the medical care area 100. In turn, the base 103a supports a surface 103b of the surgical table 102 for accommodating a patient 104. Various parameters of the surgical table 102, such as the height of the surface 103b above the floor, the orientation of the surface 103b in any direction, and the angle of the surface 103b relative to the floor, may be adjusted. In the illustrated example, the surgical table 102 further comprises adjustable restraints 103c for holding the patient 104 in a fixed position against the surgical table 102. In some examples (not depicted), the surgical table 102 further comprises one or more sensors such as orientation sensors (e.g., for detecting the angle of the surface 103b of the surgical table 102), motion sensors (e.g., for detecting changes in height and/or angle 105 of the surface 103b of the surgical table 102), pressure sensors (e.g., for detecting the placement of various body parts of the patient 104 on the surface 103b of the surgical table 102), weight sensors (e.g., for collecting weight data on the patient 104), etc. In some examples (not depicted), the surgical table 102 further comprises one or more optical units (e.g., laser units) that may be used to detect the height of the surface 103b above the floor, the orientation of the surface 103b in any direction, and/or the angle of the surface 103b relative to the floor. In some examples, a laser unit may be attached to the surgical table 102 such that raising, lowering, sliding, and/or tilting the surgical table 102 changes the direction in which the laser beam is pointed. The direction of the laser beam may be detected by one or more sensors within the medical care area 100. For example, when the surgical table 102 is not tilted, the laser beam may point at a first light sensor located on a wall of the medical care area 100. However, as the surgical table 102 is tilted, the laser beam may change direction such that it instead points at a second light sensor located on a ceiling of the medical care area 100. Based on the positions of the first light sensor, the second light sensor, the laser beam, and the surgical table 102, the angle at which the surgical table 102 is tilted may be automatically calculated. In some examples, the light sensors may additionally be located on the underside of the patient-supporting structure (e.g., the table surface) of the surgical table.

In some examples, the surgical table 102 may be a connected device that may exchange information with one or more remote devices (e.g., a cloud platform 112) via one or more networks. The information may be related to the sensor readings, the configuration of the surgical table 102 and/or the medical care area 100 throughout the surgical procedure, the surgical procedure performed on the patient 104, the outcome of the surgical procedure, the timestamps associated with various events during the surgical procedure (e.g., when an at-risk position was initiated, when it was ended, when various user inputs were provided, when various surgical milestones were reached), or the like. The information may be used for analysis and/or reporting of the surgical procedure. In some examples, the information exchanged between the surgical table 102 and the one or more remote devices may be analyzed using one or more machine learning models. For example, the information may be analyzed by a statistical model, which may determine the average, median, distributions, or the like of any parameter. In some examples, the machine learning model may identify trends and/or correlations between how the surgical procedure is conducted (e.g., the configuration of the surgical table 102, the placement of body parts of the patient 104, how long the patient stayed in a given at-risk position in total, how long the patient stayed in the at-risk position after a warning is issued, whether the user opted out of an automatic intervention) and the outcomes of patients such as the patient 104. In some examples, the machine learning model may identify optimal parameters for the surgical table 102 to maximize patient safety and minimize surgical complications.

With reference to FIG. 1A, a medical practitioner 108 is located within the medical care area 100 to care for and/or operate on the patient 104. For example, the medical practitioner 108 may be a surgeon operating on the patient 104 in person. In some examples (not depicted), the medical practitioner 108 may be located outside the medical care area 100. For example, the medical practitioner 108 may be a surgeon remotely supervising the surgical procedure and/or remotely controlling a robot to operate on the patient 104.

Before, during, and/or after a surgical procedure, the medical practitioner 108 may adjust various configurations of the medical care area 100. For example, the medical practitioner 108 may adjust various parameters, such as height and/or angle 105, of the surgical table 102. In the illustrated example, the angle 105 is defined as the angle between the surface 103b of the surgical table 102 and a horizontal level 106. In some examples, the horizontal level 106 may run parallel to the floor of the medical care area 100. In some examples, the horizontal level 106 may be an objective level irrespective of the floor level. When the surgical table 102 is tilted at the angle 105, the body position of the patient 104 changes along with the surgical table 102. For example, when the surgical table 102 is tilted at the angle 105, the head and feet of the patient 104 are also tilted at a corresponding angle. In some examples, the head of the patient 104 may be positioned lower than the feet of the patient 104, and vice versa. In some examples, the angle 105 of the surgical table 102 may put the patient 104 in an at-risk position, such as a Trendelenburg position.

The medical care area 100 further comprises a controller 109 for receiving and transmitting user inputs. To adjust the angle 105 of the surgical table 102, the medical practitioner 108 can provide one or more inputs using the controller 109. In the illustrated example, the controller 109 is a handheld device that has a wired connection to one or more objects within the medical care area 100, including the surgical table 102, but it should be appreciated that the controller 109 can alternatively communicate with the surgical table 102 via a wireless connection. In some examples, the controller 109 may include a display screen. In some examples, the controller 109 may be a user interface built into the surgical table 102, such as one or more buttons or touchscreens on the surgical table 102. In some examples, the controller 109 may be a remote controller that may wirelessly control the surgical table 102. In some examples, there may be a plurality of controllers 109, e.g., including a handheld controller, a controller built into the surgical table, and/or a wireless remote controller. In some examples, in addition to controlling the angle 105 of the surgical table 102, the controller 109 may control other parameters associated with the configuration of the medical care area 100, such as the height of the surgical table 102, movement of the surgical table 102 in any direction, deployment of restraints 103c for the patient 104, adjustment of lighting, adjustment of display settings, adjustment of audio settings, etc.

The medical care area 100 further comprises a display 110. In the illustrated example, the display 110 is a surgical display screen located inside the medical care area 100 and communicatively coupled to the surgical table 102 and/or the cloud platform 112. In some examples, the medical care area 100 can be further associated with one or more additional displays inside and/or outside the medical care area 100, such as a surgical control panel, a dashboard, a central display 116 for monitoring multiple medical care areas (e.g., multiple operating rooms in a hospital). The display 110 may convey information in visual format, auditory format, and/or haptic format.

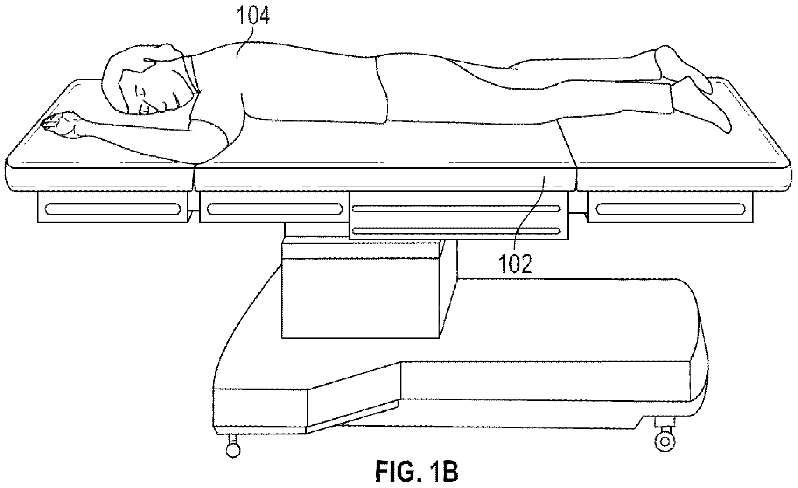

An aspect of the present disclosure is to output warnings for the medical practitioner 108 related to the patient 104 and the surgical table 102. In some examples, the medical practitioner 108 may be warned when the patient 104 has been placed in an at-risk position for an extended duration. In the illustrated example, the surgical table 102 is tilted at the angle 105 such that the patient 104 is placed in a Trendelenburg position. The Trendelenburg position is an at-risk position because the head of the patient 104 is positioned beneath the feet of the patient 104, thereby increasing blood flow to the patient's head, increasing intraocular pressure, and/or increasing intracranial pressure. This may lead to physiological changes including increased venous return, increased intra-cranial and cardiac pressure, ocular edema, and respiratory complications. These physiological changes, in turn, may increase the risk of surgical complications for the patient 104. To decrease the possibility of complications, an at-risk position such as the Trendelenburg position should be used for the shortest length of time possible. During a surgical procedure, the surgical table 102 may be returned to a horizontal or heads-up position after a certain time period has elapsed, thereby moving the patient 104 out of the at-risk position. FIGS. 1B-1D illustrate additional at-risk positions in which a patient 104 may be placed.

FIG. 1B illustrates a patient 104 placed in a prone position on a surgical table 102, such that the abdomen of the patient 104 is flush against the surface of the surgical table 102. Although the surgical table 102 is not tilted, the prone position is an at-risk position because, if maintained for an extended duration, it may lead to physiological changes including increased intra-abdominal and central venous pressure, abdominal compartment syndrome, limb compartment syndrome, nerve injuries, etc. These physiological changes, in turn, may increase the risk of surgical complications for the patient 104. In some examples, the surgical table 102 may use sensors (e.g., pressure sensors) to detect the position of the patient 104 (e.g., by determining which body parts are in contact with the surface of the surgical table 102) and determine that the patient 104 is in an at-risk position. In some examples, the medical practitioner 108 may use the controller 109 to communicate with and inform the surgical table 102 that the patient 104 is in the prone position. For example, the medical practitioner 108 may press a button on the controller 109 to indicate that the patient 104 is lying face-down (i.e., the patient's abdomen is flush against the surgical table 102). In some examples, the system may make the determination using a machine-learning model. By analyzing one or more images of the surgical environment, the machine-learning model can identify the position of the patient 104 (e.g., body parts of the patient 104, such as head, face, chest, abdomen, back, waist, hips, legs, knees, and feet of the patient 104, and/or their directions and relative positions to each other), or any combination thereof. Accordingly, the system may warn the medical practitioner 108 when the patient 104 has been in the prone position for an extended duration, as described in detail herein. In some examples, the medical practitioner 108 may be instructed to flip the patient 104 into a supine position after a certain time period has elapsed.

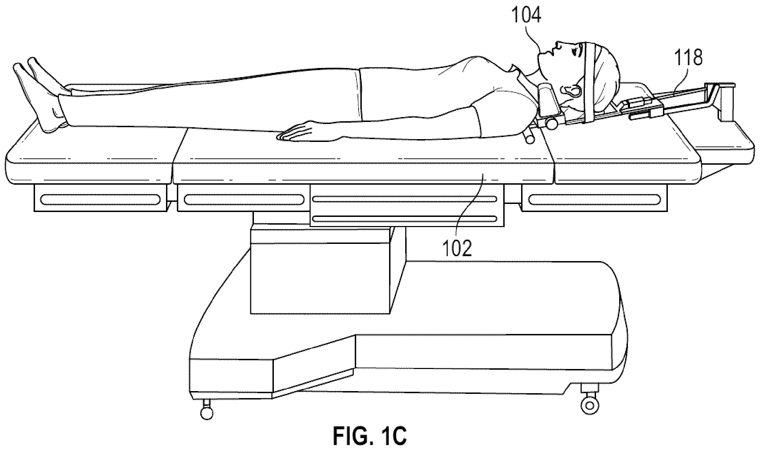

FIG. 1C illustrates a patient 104 placed in a traction position on a surgical table 102. The surgical table 102 further comprises a traction mechanism 118 for producing tension on various body parts of the patient 104. In the illustrated example, the traction mechanism 118 provides cervical traction via an attachment to the neck and head of the patient 104. Similar to the prone position of FIG. 1B, although the surgical table 102 is not tilted, the traction position is an at-risk position because, if maintained for an extended duration, it may lead to physiological changes including damage to tissues surrounding the traction mechanism 118, nerve and vascular injury from excessive traction, etc. One or more force sensors of the surgical table 102 may obtain tension measurements indicative of traction. Based on the tension measurements, the system may determine that the patient 104 is in a traction position and is thus in an at-risk position. In some examples, the system may make the determination using a machine-learning model. By analyzing one or more images of the surgical environment, the machine-learning model can identify traction mechanisms applied to the patient 104. In some examples, the system may make the determination based on one or more user inputs. When the patient 104 has been in the traction position for an extended duration, the system may warn the medical practitioner 108 to reposition the patient 104 and/or temporarily reduce the traction force applied by the traction mechanism 118.

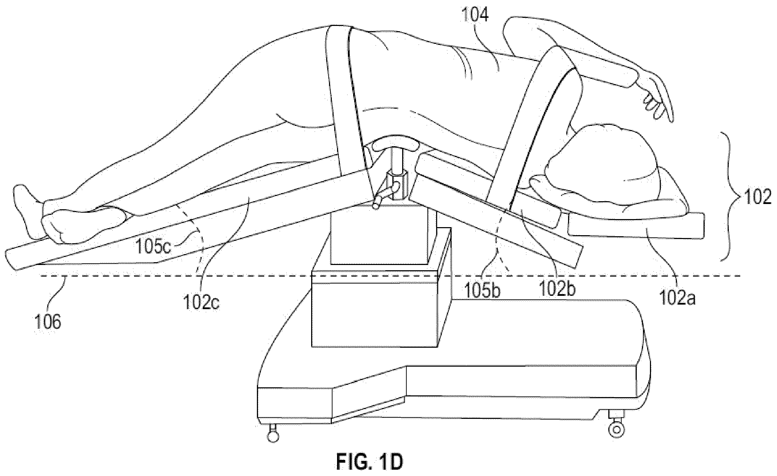

FIG. 1D illustrates a patient 104 placed in a tilt position on a surgical table 102. The surgical table 102 comprises several independently tiltable table sections: a first table section 102a, a second table section 102b, and a third table section 102c, as shown in the illustrated example. The surgical table 102 may thus be tilted in sections such that various body parts of the patient 104 may be elevated during the surgical procedure. For example, the patient 104 may be placed lying sideways on the surgical table 102. The head of the patient 104 may be flush against the first table section 102a, which may not be tilted relative to the horizontal level 106. One shoulder of the patient 104 may be flush against the second table section 102b, which may be tilted at an angle 105b relative to the horizontal level 106; likewise, one hip of the patient 104 may be flush against the third table section 102c, which may be tilted at an angle 105c relative to the horizontal level 106. The angle 105b and the angle 105c may be selected such that the waist of the patient 104 is elevated relative to the rest of the body. This tilt position allows the medical practitioner 108 to access certain body regions of the patient 104 that would be inaccessible in other positions. However, the tilt position is an at-risk position because, if maintained for an extended duration, it may lead to physiological changes such as muscle and nerve injuries. The system can determine that the patient 104 is in the depicted at-risk position using any combination of techniques described herein. When the patient 104 has been in the tilt position for an extended duration, the system may warn the medical practitioner 108 to reposition the patient 104 and/or return the surgical table 102 to a horizontal or heads-up position after a certain time period has elapsed.

To automatically track the elapsed time period during which the patient 104 is in an at-risk position (e.g., the positions shown in FIGS. 1A-D), the surgical table 102 uses a timing mechanism. In some examples, the timing mechanism may be a timer integrated into the surgical table 102. In some examples, the timing mechanism may be a separate hardware component external to and communicatively coupled to the surgical table 102. In some examples, the timing mechanism can be either built-in or retrofitted into either the surgical table 102 or the separate hardware component. In some examples, the timing mechanism can be a software timer program comprising instructions that when executed, tracks elapsed time. The instructions can be stored in a local memory unit integrated with the surgical table 102, or on one or more remote devices (e.g., cloud platform 112).

With reference to FIG. 1A, based on the measurements of the timer, the system may issue warnings for the medical practitioner 108. In response to the warnings, the medical practitioner 108 may use the controller 109 or another input device to provide a user input to the surgical table 102 (e.g., ignore the warnings for 30 minutes, or change the angle 105 of the surgical table 102). The user input may be stored and analyzed to improve future clinical protocols and practices, as described herein. The warnings may be displayed to users (e.g., the medical practitioner 108) through various means, both within and outside the medical care area 100. In some examples, at least one of the warnings may be displayed on the display 110 within the medical care area 100. In some examples, at least one of the warnings may be displayed on the central display 116 outside of the medical care area 100. In the illustrated example, central display 116 is a dashboard located in a central control room for monitoring multiple medical care areas, including the medical care area 100. In some examples, at least one of the warnings may be displayed on at least one personal device 114, which may comprise a mobile phone, a pager, a radio, etc. The personal device 114 may be located either within or outside the medical care area 100. In some examples, the warnings may be transmitted to and stored by the cloud platform 112 implemented by one or more remote electronic devices. The cloud platform 112 may store information associated with surgical procedure and, in some examples, may transmit the information to an analytics platform for analysis.

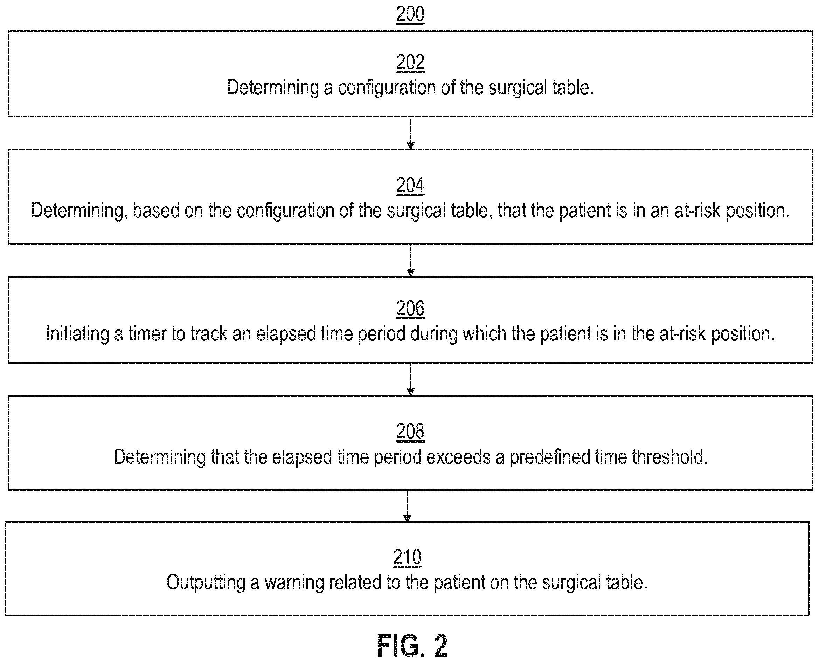

FIG. 2 illustrates an exemplary process 200 for outputting warnings related to a patient placed on a surgical table (e.g., the patient 104 on the surgical table 102 of FIGS. 1A-1D), according to various examples. Process 200 is performed, for example, using one or more electronic devices implementing a software platform. In some examples, process 200 is performed using a client-server system, and the blocks of process 200 are divided up in any manner between the server and a client device. In other examples, the blocks of process 200 are divided up between the server and multiple client devices. In other examples, process 200 is performed using only a client device or only multiple client devices. In process 200, some blocks are, optionally, combined, the order of some blocks is, optionally, changed, and some blocks are, optionally, omitted. In some examples, additional steps may be performed in combination with the process 200. Accordingly, the operations as illustrated (and described in greater detail below) are exemplary by nature and, as such, should not be viewed as limiting.

At block 202, an exemplary system (e.g., one or more electronic devices) determines a configuration of the surgical table, such as the angle of the surgical table relative to a horizontal level, the height of the surgical table above the floor of a medical care area, the movement of the surgical table, the placement of body parts of the patient relative to the surgical table (e.g., which direction the patient is facing), the deployment of any restraints, supports, and/or mechanisms of the surgical table (e.g., a traction mechanism), the orientation of different parts of the surgical table, or the like. The system can collect data used to determine the configuration of the surgical table though a variety of techniques, as described below.

In some examples, the system may collect data from a medical practitioner. The medical practitioner may provide inputs via a control device (e.g., controller 109 of FIG. 1A) communicatively coupled with the surgical table. The control device may include one or more buttons, displays, touchscreens, etc. that enables the medical practitioner to communicate with the surgical table. In some examples, based on the medical practitioner's inputs when setting up the surgical table (e.g., which buttons were pressed to set the height of the surgical table), the system may determine the configuration of the surgical table. In some examples, the medical practitioner may manually input information about the configuration of the surgical table into the system (e.g., which direction the patient is facing).

In some examples, the system may include one or more sensors that collect data. The one or more sensors may collect data with medical practitioner input (e.g., the medical practitioner uses the control device to activate a specific sensor) or without medical practitioner input (e.g., certain sensors automatically activate during a surgical procedure). For example, the system may automatically activate cameras when a medical practitioner is present inside the medical care area, and the cameras may collect image data about the position of the medical practitioner and/or the patient within the medical care area. The one or more sensors may include cameras disposed throughout the surgical environment (e.g., for collecting image data to be used by one or more machine-learning models described herein), gyroscopes and/or orientation sensors (e.g., for detecting the height and/or angle of the surgical table), motion sensors (e.g., for detecting the movements of the surgical table and/or patient), pressure sensors (e.g., for detecting the placement of various body parts of the patient on the surgical table), weight sensors (e.g., for collecting weight data on the patient), force sensors (e.g., for measuring how much force is applied to a body part of the patient), distance sensors (e.g., for detecting distances between the medical practitioner and the surgical table), light sensors, temperature sensors, etc. In some examples, the one or more sensors may be integrated into the surgical table. In some examples, the one or more sensors may be separate hardware components external to and communicatively coupled to the surgical table.

In some examples (e.g., to identify whether the configuration of the surgical table is associated with a Trendelenburg position and/or another at-risk tilt position), the system may determine an angle of the surgical table relative to a horizontal level. In some examples, the angle of the surgical table may be retrieved from a memory unit associated with the surgical table. In some examples, the angle of the surgical table may be determined based on user input(s), such as a user's previous button presses on a remote controller to tilt the table. In some examples, the angle of the surgical table may be determined based on one or more sensors.

In some examples, a machine-learning model may be used to provide additional detail about the configuration of the surgical table. By analyzing one or more images of the surgical environment, the machine-learning model can measure the angle between the table surface of the surgical table and a horizontal level, the movement of the surgical table, the position of the patient (e.g., body parts of the patient, such as head, face, chest, abdomen, back, waist, hips, legs, knees, and feet of the patient, and their relative positions to each other), or any combination thereof. For example, one or more sensors may be used to determine that an angle of the surgical table is +/-30 degrees, with +30 degrees referring to the configuration in which the patient's head is higher than the patient's feet and -30 degrees referring to the configuration in which the patient's head is lower than the patient's feet. However, in some examples, the one or more sensors alone may not be able to determine which end of the surgical table that the patient's head is resting on, and thus, may not be able to determine whether the angle is +30 degrees or -30 degrees. To account for this, the trained machine learning model may be used to determine the placement of body parts of the patient on the surgical table. For example, the trained machine learning model may determine that the patient's head is lower than the patient's feet; accordingly, the angle of the surgical table may be determined to be -30 degrees.

In some examples (e.g., to identify whether the configuration of the surgical table is associated with a prone position), the system may determine which body parts of the patient are in contact with the surgical table. In some examples, the system may make the determination based on readings from one or more pressure sensors of the surgical table (e.g., by determining which body parts are in contact with the surface of the surgical table). In some examples, the system may make the determination using a machine-learning model. By analyzing one or more images of the surgical environment, the machine-learning model can identify which body parts of the patient (e.g., head, face, chest, abdomen, back, waist, hips, legs, knees, feet of the patient, and the like) are in contact with the surgical table. In some examples, the system may make the determination based on one or more user inputs (e.g., a user input indicative that the patient has been placed in the prone position).

In some examples (e.g., to identify whether the configuration of the surgical table is associated with a traction position), the system may determine whether any restraints, supports, and/or mechanisms of the surgical table are in use. For example, the system may detect whether a traction mechanism is in use. Collecting data to determine whether the traction mechanism is in use involves collecting data about how the traction mechanism is attached to the patient, what body part the traction mechanism is attached to, how much mechanical tension is being applied to the body part, etc. In some examples, the user may provide the system with information about the restraints, supports, and/or mechanisms using a control device. In some examples, a camera and/or force sensor may take measurements used to determine whether any restraints, supports, and/or mechanisms of the surgical table are in use. In some examples, the system may make the determination using a machine-learning model. By analyzing one or more images of the surgical environment, the machine-learning model can identify traction mechanisms applied to the patient.

At block 204, the system determines, based on the configuration of the surgical table (as determined in block 202), that the patient is in an at-risk position. An at-risk position may include any patient position that can increase the risk of surgical complications. In some examples, the definitions for what constitutes an at-risk position may be programmed into the system by default. In some examples, a user of the system (e.g., a medical practitioner) may set a custom definition for what constitutes an at-risk position.

In some examples, the at-risk position may include a Trendelenburg position, a prone position, a traction position, or a tilt position. A Trendelenburg position may be any position in which the head of the patient is positioned significantly lower than the feet of the patient. To determine if the patient is in a Trendelenburg position, the system determines if the angle of the surgical table (as determined in block 202) exceeds a threshold value. For example, the system may compare an angle between a table surface of the surgical table (e.g., the surface for accommodating the patient) and a horizontal level (e.g., the level of the floor of the medical care area) to a predefined angle threshold. In some examples, the predefined angle threshold may be an angle used to define a Trendelenburg position, e.g., when the surgical table is tilted beyond the angle, the patient enters the Trendelenburg position. The predefined angle threshold may be equal to or greater than, for example: 5 degrees, 10 degrees, 15 degrees, 20 degrees, 25 degrees, 30 degrees, 35 degrees, or any value in between. In some examples, a regular Trendelenburg position may be achieved by tilting the surgical table at an angle between 15 to 29 degrees. In some examples, an extreme Trendelenburg position may be achieved by tilting the surgical table at an angle exceeding 29 degrees.

The at-risk position may include other tilt positions, in which the surgical table is tilted such that any part of the patient's body is elevated relative to the other parts. To determine if the patient is in a tilt position, the system may perform the same angle determination described above for the Trendelenburg position. Furthermore, in some tilt positions, the surgical table may comprise several tiltable table sections, which may be independently tilted such that various body parts of the patient may be elevated during the surgical procedure. In such examples, to determine if the patient is in a tilt position, the system may determine if the angle of one or more of the tiltable table sections exceeds a predefined angle threshold. For example (as described in FIG. 1D), a first tiltable table section may be tilted at a first angle, and a second tiltable table section may be tilted at a second angle such that the waist of the patient is elevated relative to the rest of the body. This tilt position allows the medical practitioner to access certain body regions of the patient that would be inaccessible otherwise. If one or both of the first angle and the second angle exceed a predefined angle threshold (e.g., 15 degrees), the patient may be considered to be in an at-risk position.

In some examples, the at-risk position does not involve any tilting of the surgical table, but instead involves the position of the patient. For example, the at-risk position may be a prone position, in which the surgical table is not tilted and the patient's abdomen is flush against the surgical table. To determine if the patient is in the prone position, the system may examine the data collected in block 202 as described above. In some examples, the patient's position may be affected by one or more restraints, supports, and/or mechanisms of the surgical table that cause the patient to be in an at-risk position. For example, the at-risk position may be a traction position, in which mechanical tension is applied to one or more body parts of the patient via a traction mechanism. To determine if the patient is in the traction position, the system may examine the data collected in block 202 as described above. In particular, the system may determine if the mechanical tension applied by the traction mechanism (as determined in block 202) exceeds a threshold value.

At block 206, the system initiates a timer to track an elapsed time period during which the patient is in the at-risk position (as determined in block 204). In some examples, the timing mechanism may be a timer integrated into the surgical table 102. In some examples, the timing mechanism may be a separate hardware component external to and communicatively coupled to the surgical table 102. In some examples, the timing mechanism can be either built-in or retrofitted into either the surgical table 102 or the separate hardware component. In some examples, the timing mechanism can be a software timer program comprising instructions that when executed, tracks elapsed time. The instructions can be stored in a local memory unit integrated with the surgical table 102, or on one or more remote devices (e.g., cloud platform 112).

At block 208, the system determines that the elapsed time period exceeds a predefined time threshold. The predefined time threshold may be the maximum duration of time (e.g., 30 minutes) a patient can spend in an at-risk position without a risk of surgical complications. If a patient's time in the at-risk position exceeds the predefined time threshold, the patient may be at greater risk of surgical complications. In some examples, the value may be a default value based on guidelines and/or recommendations from standard-setting organizations. In some examples, the value of the predefined time threshold, and optionally any other thresholds used in the process 200 (e.g., angle threshold), may be customized based on various factors associated with the patient, the medical practitioner, and/or the surgical procedure, as described below.

In some examples, the value of the predefined time threshold can vary based on individual patients. In some examples, the predefined time threshold may be determined based on a Body Mass Index (BMI) of the patient. For example, a patient with a higher BMI may be assigned a shorter time threshold than a patient with a lower BMI because patients with higher BMIs tend to experience surgical complications more frequently or sooner during a given surgical procedure. In some examples, the predefined time threshold may be determined based on an age of the patient. For example, an older patient may be assigned a shorter time threshold than a younger patient because older patients tend to experience surgical complications more frequently or sooner during a given surgical procedure. In some examples, the predefined time threshold may be determined based on historical data associated with the patient, such as surgical history, allergies, and/or pre-existing medical conditions of the patient. For example, a patient with a blood clotting disorder and a history of surgical complications may be assigned a shorter time threshold than a patient without such a history. As a further example, a patient that is hypertensive may be assigned a shorter time threshold than a patient that is not.

In some examples, the value of the predefined time threshold can vary based on the type of surgical procedure being performed. For example, patients undergoing a pelvic operation may avoid complications when the duration of time spent in a 30-degree Trendelenburg position does not exceed a first time threshold, whereas patients undergoing a spinal cord operation may need to be restricted to a second time threshold to avoid complications.

In some examples, the value of the predefined time threshold can vary based on historical data. As discussed above, historical data related to a plurality of past surgical procedures may be analyzed by a statistical model, which may determine the average, median, distributions, or the like of any parameter. In some examples, the machine learning model may identify trends and/or correlations between how the surgical procedure is conducted (e.g., the configuration of the surgical table, the position of the patient, the associated time stamps) and the outcomes of patients. In some examples, the machine learning model may identify optimal parameters, including the predefined time threshold, for the surgical table to maximize patient safety and minimize surgical complications.

In some examples, the value of the predefined time threshold may vary based on a milestone and/or phase associated with the surgical procedure. A surgical milestone and/or phase may indicate the stage of progression through a surgical procedure. The plurality of predefined milestones may include: whether an operating room is ready, whether operating room setup has started, whether a medical staff member (e.g., the medical practitioner, the scrub nurse, the technician) is donning surgical attire (e.g., masks, gloves, caps, gowns), whether operating room equipment is being set up, whether the patient is brought in to the operating room, whether the patient is ready for intubation or anesthesia, whether a timeout is occurring, whether the timeout has occurred, whether the patient is intubated or anesthetized, whether the patient has been prepped and draped for surgery, whether the patient is ready for surgery, whether a surgery site prep is complete, whether a surgery has started, whether a surgical count is occurring, whether the surgery is closing, whether a dressing is applied to the patient, whether the surgery is stopped, whether the patient is brought out of the operating room, whether the operating room is being cleaned, whether the operating room is clean, or any combination thereof. It should be understood that the foregoing list of milestones is merely exemplary. There may be fewer, additional, or different predefined milestones, for instance, depending on a type of surgical procedure.

To analyze and determine the milestones of the surgical procedure, the system may input one or more images from a camera into a computer vision algorithm (e.g., a trained machine-learning model) configured to determine the milestones of the surgical procedure. For example, the system may use the one or more trained machine learning models to detect one or more objects or events, which are in turn used to determine the one or more surgical milestones (e.g., surgical time points, surgical phases). The one or more trained machine learning models may include an object detection algorithm, an object tracking algorithm, a video action detection algorithm, an anomaly detection algorithm, or any combination thereof.

In some examples, the camera may capture images of people and objects within the medical care area. In some examples, the camera may be installed on one or more surgical devices and capture close-up images of the surgical procedure. The system may detect one or more surgical milestones using one or more trained machine-learning models based on the received one or more images. By utilizing images captured by cameras generally installed in the medical care area in conjunction with information from surgical devices, the system may provide a more accurate and realistic identification of surgical milestones and estimation of time between surgical milestones (e.g., surgery started, surgery closing). For example, a cholecystectomy may last anywhere from one to three hours (or even longer), depending on the patient, the anatomy, and the surgeon's skills. Therefore, being able to correctly identify all the surgical milestones specific to this procedure (using intra-operative endoscopic images among other types of images) may result in a more accurate overall remaining time estimation and allow the system to determine an appropriate value for the predefined time threshold. Additional details related to detection of surgical milestones can be found in U.S. Patent Application No. 18/334,342, filed June 13, 2023, and entitled, "SYSTEMS AND METHODS FOR MONITORING SURGICAL WORKFLOW AND PROGRESS" (Attorney Docket No.: 16890-20044.00), which is incorporated herein by reference.

In some examples, the value of the predefined time threshold can vary based on the medical staff (e.g., medical practitioners, nurses) associated with the surgical procedure. In some examples, a user profile (e.g., created based on the subjective preferences of the user and past surgical performances) associated with the medical practitioner performing the surgical procedure may be used to determine the predefined time threshold. The user profile can comprise information related to one or more users (e.g., an individual user, a group of users). For example, the identified user profile may comprise an identity of the user (e.g., a surgeon, a medical staff member, or an administrator), one or more specialties of the user, one or more device settings specific to the user, one or more procedure types specific to the user, or any combination thereof. The one or more device settings may be based on settings used by the user in previous surgical procedures, preferences or configurations indicated by the user, or any combination thereof. The one or more procedure types may include procedure types that the user can perform (e.g., based on their specialty, based on their qualifications) and/or has performed in previous surgical procedures. A user profile may include a profile of an individual or a group of individuals associated with the surgical procedure. In some examples, the user profile can include a surgeon profile, a medical staff profile, an administrator profile, or any combination thereof.

In some examples, the value of the predefined time threshold can vary based on the type of at-risk position that the patient is in. For example, the predefined time threshold may be shorter for a patient in a prone position than in a Trendelenburg position. In some examples, the value of the predefined threshold can vary based on how extreme the at-risk position is. For example, a regular Trendelenburg position may be achieved by tilting the surgical table at an angle between 15 to 29 degrees, and an extreme Trendelenburg position may be achieved by tilting the surgical table at an angle exceeding 29 degrees. The predefined time threshold may be shorter for a patient in an extreme Trendelenburg position than a patient in a regular Trendelenburg position because patients in extreme positions tend to experience surgical complications more frequently during a given surgical procedure than patients in less extreme positions.

In some examples, the system may input the various factors above (patient data such as BMI, age, surgical history, allergies, and/or pre-existing medical conditions; the type of surgical procedure; historical trends and/or success rates associated with the surgical procedure; the milestone and/or phase of the surgical procedure; the user profile of the medical practitioner; the type and extremity of the at-risk position, or the like) into a trained machine learning model to determine the predefined time threshold. In some examples, the trained machine learning model may be trained on aggregated historical medical data (e.g., from previously performed surgical procedures). The trained machine learning model may include a prediction algorithm that outputs, e.g., an appropriate maximum duration that a patient can spend in an at-risk position without increased risk of surgical complications. The output of the trained machine learning model may be used to determine the predefined time threshold used in block 208.

At block 210, the system outputs a warning related to the patient on the surgical table. The system may display the warning and, optionally, the elapsed time period (as determined in block 206) on one or more displays within or outside of the medical care area. The one or more displays may comprise a display on a control device (e.g., controller 109 of FIG. 1A) communicatively coupled with the surgical table. The one or more displays may comprise a surgical display, a surgical control panel, a dashboard (e.g., in the medical care area, in the hallway outside the medical care area, in a central control room), etc. The warning and/or the elapsed time period may be conveyed by the one or more displays in visual format, auditory format, haptic format, or any combination thereof.

In some examples, the warning and/or the elapsed time period may be displayed in accordance with a color scheme to indicate the severity of the warning and/or the length of the elapsed time period. The color scheme may indicate how close the elapsed time is to reaching the predefined time threshold and/or whether the elapsed time has exceeded the predefined time threshold. For example, when the elapsed time period is within 10 minutes of a predefined time threshold of 30 minutes), a warning color of yellow may be displayed to indicate that the patient should be repositioned soon. In another example, when the elapsed time period is within 5 minutes of the predefined time threshold of 30 minutes), an urgent warning color of orange may be displayed to indicate that the patient should be repositioned imminently. Once the elapsed time period has exceeded the predefined time threshold, a severe warning color (e.g., red) may be displayed to indicate that the patient is overdue for repositioning.

The system may take granular time measurements for data analysis and reporting purposes. For example, the system may take measurements of how much time the patient spends in various positions (e.g., safe positions, at-risk positions, etc.) and in what order the position changes occur. In some examples, the timing mechanism (as described in block 206) comprises multiple timers for obtaining the granular time measurements. For example, the system may determine whether the angle of the surgical table is larger than a first predefined angle threshold (e.g., corresponding to the threshold for entering a regular Trendelenburg position) and/or a second predefined angle threshold (e.g., corresponding to the threshold for entering an extreme Trendelenburg position), then use different timers to track the elapsed time period that the patient spends in each position. The system may use a first timer to measure how long a patient spends in a non-tilted (i.e., horizontal level) position, a second timer to measure how long the patient spends in a regular Trendelenburg position, and a third timer to measure how long the patient spends in an extreme Trendelenburg position. The system may record data associated with the timers. The system may further record data associated with the chronological order of the positions and the outcome of the surgical procedure performed. For example, the system may record that the patient spent 10 minutes in a regular Trendelenburg position, then 7 minutes in an extreme Trendelenburg position, then 30 minutes in a non-tilted position, and experienced positive health outcomes following the surgery. This data may be recorded for each surgical procedure and may be stored by one or more memory components of the system. In some examples, the data may be transmitted to one or more cloud platforms to be stored and analyzed. In some examples, the data may be documented in electronic health records (EMRs) or other patient records.

If a warning is issued (as described in block 210) and the patient remains in the at-risk position, the system may eventually issue another warning and/or provide an automatic intervention (e.g., returning the surgical table to a horizontal position). The automatic intervention may require preparation to ensure patient safety (e.g., the medical practitioner may restrain the patient on the surgical table and/or clear the surroundings of hazards). As such, additional warnings may be provided in advance of the automatic intervention to give the user sufficient notice and allow the user to opt out of the intervention. In some examples, the warning(s) may include a countdown to when the automatic intervention will occur, a description of what the automatic intervention will entail, instructions about how to prepare for the automatic intervention, etc. The countdown may be of a sufficient duration to ensure that the medical practitioner has enough time to prepare the surgical table for the automatic intervention, such as removing instrumentation surrounding the surgical table, restraining the patient on the surgical table, stepping away from the surgical table, etc. In some examples, via the control device, the user may provide a user input to the system to opt out of performing the automatic intervention (e.g., the medical practitioner may decide to opt out of repositioning the surgical table in order to finish performing the surgical procedure).

In some examples, the system may output warnings based on the angle of the surgical table. For example, if the angle between the table surface of the surgical table and the horizontal level exceeds a first predefined angle threshold (e.g., 15 degrees or greater), the system may warn the medical practitioner when the elapsed time period exceeds a first predefined time threshold associated with the first predefined angle threshold. If the angle further exceeds a second predefined angle threshold (e.g., 30 degrees or greater), the system may warn the medical practitioner sooner (e.g., when the elapsed time period exceeds a second predefined time threshold associated with the second predefined angle threshold that is shorter than the first predefined time threshold).

In some examples, the system may output warnings based on the elapsed time period (as described in block 206) and/or whether the elapsed time period exceeds a predefined time threshold. In some examples, the system may determine that the elapsed time period exceeds multiple predefined time thresholds and output multiple warnings. For example, the system may output a first warning when a first predefined time threshold is exceeded (e.g., 30 minutes) and a second warning after a second predefined time threshold is exceeded (e.g., 60 minutes). The second predefined time threshold may be measured relative to the start time of the elapsed time period (e.g., the start of the surgical procedure) and/or relative to the first predefined time threshold (e.g., 15 minutes after the first predefined time threshold). In some examples, the second warning may comprise a countdown notification for a given time frame, such as a countdown from 100 seconds to 0 seconds. In some examples, the second warning may comprise a notification that the surgical table will automatically return to a non-tilted position within a given time frame, such as the time frame of the countdown notification.

In some examples, the system may further perform one or more actions after outputting the second (or first) warning. The one or more actions may include an automatic intervention, such as automatically levelling the surgical table, deactivating the traction mechanism, etc. In some examples, the system may further receive a user request (e.g., from the medical practitioner) to prevent the automatic intervention from occurring. Upon receiving the user request, the system may forego the automatic intervention. The user request may be provided via a control device (e.g., a user interface associated with the surgical table). Data associated with the user request, such as the timing and nature of the user request, may be stored by one or more memory components of the system. In some examples, the user request data may be transmitted to one or more cloud platforms to be stored and analyzed. For example, repeated opt-outs by a user may be identified and reported.

To ensure patient safety, the system may determine whether it is appropriate to perform the automatic intervention and how the user may prepare for the automatic intervention. In some examples, the system may utilize the computer vision algorithm (as described in block 204) to determine whether it is appropriate to perform an automatic action associated with the surgical table. For example, to determine whether the conditions within the medical care area are safe enough to automatically level the surgical table, the system may input one or more images from cameras within the medical care area into the computer vision algorithm. Based on the images, the system may determine, for example, if any instruments are obstructing the predicted path of the surgical table, the location of the medical practitioners within the medical care area, steps to take to prepare the medical care area for the automatic intervention, etc.

Upon completing the automatic intervention, the system may display the elapsed time period that the patient has spent under the new conditions following the automatic intervention (e.g., how long the patient has spent in the non-tilted position after the surgical table is automatically levelled). Based on the elapsed time period, the system may determine what an appropriate time to return the patient to the at-risk position will be. The appropriate time to return the patient to the at-risk position may vary across different patients. For example, patients with pre-existing health conditions may require more time in the non-tilted position before returning to the at-risk position than other patients without pre-existing health conditions. As another example, older patients may require more time in the non-tilted position before returning to the at-risk position than younger patients. In some examples, the system may further automatically return the patient to the at-risk position after a set period of time has elapsed (e.g., 5 minutes after levelling the surgical table). In some examples, the system may notify the user of the return time and the at-risk position (e.g., informing the medical practitioner that the surgical table will return to the Trendelenburg position in 3 minutes) and allow the user to override the return.

As described above, the system may export data pertaining to the surgical procedure (e.g., the type of procedure, the repositioning of the surgical table, the user profile of the medical practitioner, the medical history of the patient, or the like) to a database. Data from multiple historical surgical procedures may be aggregated in the same database, forming a database of historical medical data. In some examples, the historical medical data may be analyzed to identify trends associated with various parameters of the surgical procedure as described herein. For example, the historical medical data may be analyzed by a machine-learning model and/or statistical techniques to draw correlations between the elapsed time period that the patient spends in an at-risk position and the patient outcome (e.g., occurrence of surgical complications). The trends identified from the historical medical data may be used to determine thresholds (e.g., predefined time thresholds and predefined angle thresholds), which can be used to improve surgical protocols and practices. In some examples, the data pertaining to the surgical procedure, the historical medical data, and/or the identified trends and best practices may be included in a report.

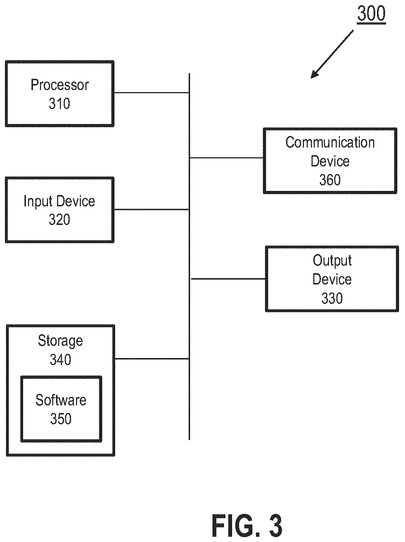

The operations described herein are optionally implemented by components depicted in FIG. 3. FIG. 3 illustrates an example of a computing device in accordance with one example. Device 300 may be a host computer connected to a network. Device 300 may be a client computer or a server. As shown in FIG. 3, device 300 may be any suitable type of microprocessor-based device, such as a personal computer, workstation, server, or handheld computing device (portable electronic device) such as a phone or tablet. The device may include, for example, one or more of processor 310, input device 320, output device 330, storage 340, and communication device 360. Input device 320 and output device 330 may generally correspond to those described above and may either be connectable or integrated with the computer.

Input device 320 may be any suitable device that provides input, such as a touch screen, keyboard or keypad, mouse, or voice-recognition device. Output device 330 may be any suitable device that provides output, such as a touch screen, haptics device, or speaker.

Storage 340 may be any suitable device that provides storage, such as an electrical, magnetic, or optical memory including a RAM, cache, hard drive, or removable storage disk. Communication device 360 may include any suitable device capable of transmitting and receiving signals over a network, such as a network interface chip or device. The components of the computer may be connected in any suitable manner, such as via a physical bus or wirelessly.

Software 350, which may be stored in storage 340 and executed by processor 310, may include, for example, the programming that embodies the functionality of the present disclosure (e.g., as embodied in the devices as described above).

Software 350 may also be stored and/or transported within any non-transitory computer-readable storage medium for use by or in connection with an instruction execution system, apparatus, or device, such as those described above, that may fetch instructions associated with the software from the instruction execution system, apparatus, or device and execute the instructions. In the context of this disclosure, a computer-readable storage medium may be any medium, such as storage 340, that may contain or store programming for use by or in connection with an instruction execution system, apparatus, or device.

Software 350 may also be propagated within any transport medium for use by or in connection with an instruction execution system, apparatus, or device, such as those described above, that may fetch instructions associated with the software from the instruction execution system, apparatus, or device and execute the instructions. In the context of this disclosure, a transport medium may be any medium that may communicate, propagate, or transport programming for use by or in connection with an instruction execution system, apparatus, or device. The transport readable medium may include, but is not limited to, an electronic, magnetic, optical, electromagnetic, or infrared wired or wireless propagation medium.

Device 300 may be connected to a network, which may be any suitable type of interconnected communication system. The network may implement any suitable communications protocol and may be secured by any suitable security protocol. The network may comprise network links of any suitable arrangement that may implement the transmission and reception of network signals, such as wireless network connections, T1 or T3 lines, cable networks, DSL, or telephone lines.

Device 300 may implement any operating system suitable for operating on the network. Software 350 may be written in any suitable programming language, such as C, C++, Java, or Python. In various examples, application software embodying the functionality of the present disclosure may be deployed in different configurations, such as in a client/server arrangement or through a Web browser as a Web-based application or Web service, for example.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims.

The foregoing description, for purpose of explanation, has been described with reference to specific examples. However, the illustrative discussions above are not intended to be exhaustive or to limit the invention to the precise forms disclosed. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

## Claims

1. A computer-implemented method for outputting warnings related to a patient on a surgical table during a surgical procedure, comprising:
determining a configuration of the surgical table;
determining, based on the configuration of the surgical table, that the patient is in an at-risk position;
initiating a timer to track an elapsed time period during which the patient is in the at-risk position;
determining that the elapsed time period exceeds a predefined time threshold; and
outputting a warning related to the patient on the surgical table.

2. The method of claim 1, wherein the at-risk position includes a Trendelenburg position, a prone position, a tilt position, a traction position, or any combination thereof.

3. The method of claim 1 or 2, wherein determining the configuration of the surgical table comprises determining if an angle between a table surface of the surgical table and a horizontal level is larger than a first predefined angle threshold.

4. The method of any one of claims 1-3, wherein the timer is a first timer, further comprising:
while the patient is in the at-risk position, determining that the angle between the table surface of the surgical table and the horizontal level is larger than a second predefined angle threshold, the second predefined angle threshold larger than the first predefined angle threshold; and
initiating a second timer.

5. The method of claim 4, wherein the first predefined angle threshold is a value ranging from 15 to 29 degrees, and wherein the second predefined angle threshold is a value larger than 29 degrees.

6. The method of any one of the preceding claims, wherein the predefined time threshold is determined based on a Body Mass Index (BMI) of the patient, an age of the patient, one or more pre-existing medical conditions of the patient, historical data associated with the patient, historical data associated with the surgical procedure, a milestone associated with the surgical procedure, a phase associated with the surgical procedure, or any combination thereof.

7. The method of any one of the preceding claims, wherein the predefined time threshold is determined based on a user profile associated with the surgical procedure, based on a type of the surgical procedure, and/or based on a machine-learning model.

8. The method of any one of the preceding claims, further comprising: displaying the elapsed time period and/or the warning on one or more displays.

9. The method of claim 8, wherein the elapsed time period is displayed in accordance with a color scheme to indicate the length of the elapsed time period.

10. The method of claim 8 or 9, wherein the one or more displays comprise a display on a control device communicatively coupled with the surgical table, a surgical display, a surgical control panel, a dashboard, or any combination thereof.

11. The method of any one of the preceding claims, wherein the warning is visual, auditory, haptic, or any combination thereof.

12. The method of any one of the preceding claims, wherein the predefined time threshold is a first predefined time threshold, the method further comprising:
determining that the elapsed time period exceeds a second predefined time threshold, the second predefined time threshold longer than the first predefined time threshold;
causing output of a notification, e.g. including a countdown, that the surgical table will automatically return to a horizontal level position; and
optionally after outputting the notification, automatically returning the surgical table to the horizontal level position.

13. The method of claim 12, further comprising:
receiving a user request to prevent the surgical table from automatically returning to the horizon level position; and
upon receiving the user request, foregoing automatically returning the surgical table to the horizontal level position.

14. A system for outputting warnings during a surgical procedure, comprising:
one or more processors;
a memory; and
one or more programs, wherein the one or more programs are stored in the memory and configured to be executed by the one or more processors, the one or more programs including instructions for:
determining a configuration of a surgical table accommodating a patient;
determining, based on the configuration of the surgical table, that the patient is in an at-risk position;
initiating a timer to track an elapsed time period during which the patient is in the at-risk position;
determining that the elapsed time period exceeds a predefined time threshold; and
outputting a warning related to the patient on the surgical table.

15. A computer program product comprising software code portions, which when executed by one or more processors of an electronic device, cause the electronic device to perform any of the methods of claims 1-13.
